# EUROPÄISCHE PATENTSCHRIFT

(11) **EP 2 130 545 B1**
(45) Veröffentlichungstag und Bekanntmachung des Hinweises auf die Patenterteilung: **22.10.2014**
(21) Anmeldenummer: 08020630.3
(22) Anmeldetag: 27.11.2008
(51) Int. Cl.: A61K 36/28, A61K 36/53, A61K 36/534, A61K 36/61, A61K 36/886, A61K 36/889, A61K 38/05, A61K 35/64, A61K 9/00, A61Q 11/00, A61K 8/92, A61K 8/97, A61K 8/98

(54) **Lösung und Tinktur**
Solution and tincture
Solution et teinture

(30) Priorität: 02.06.2008 DE 202008007376 U
(43) Veröffentlichungstag der Anmeldung: 09.12.2009
(73) Patentinhaber: Tenspolde, Thomas, Dr., 4500 Solothurn (CH)
(72) Erfinder: Tenspolde, Thomas, Dr., 4500 Solothurn (CH)
(74) Vertreter: Lenzing Gerber Stute

(56) Entgegenhaltungen:
- WO-A2-2006/109344
- TAN S K ET AL: "Comparing the safety, tolerability and efficacy of biotene mouthwash, aloe vera and manuka honey juice for the prophylaxis of oral mucositis" BONE MARROW TRANSPLANTATION, Bd. 41, Nr. Suppl. 1, März 2008 (2008-03), Seite S402, XP002558096 & 34TH ANNUAL MEETING OF THE EUROPEAN-GROUP-FOR-BLOOD-AND-MARROW-TRANSP LANTATION/24ND MEETING OF THE E; FLORENCE, ITALY; MARCH 30 -APRIL 02, 2008 ISSN: 0268-3369
- ROSENDALE DOUGLAS I ET AL: "High-throughput microbial bioassays to screen potential New Zealand functional food ingredients intended to manage the growth of probiotic and pathogenic gut bacteria" INTERNATIONAL JOURNAL OF FOOD SCIENCE & TECHNOLOGY, Bd. 43, Nr. 12, Dezember 2008 (2008-12), Seiten 2257-2267, XP002558097 ISSN: 0950-5423
- MAVRIC ELVIRA ET AL: "Identification and quantification of methylglyoxal as the dominant antibacterial constituent of Manuka (Leptospermum scoparium) honeys from New Zealand" MOLECULAR NUTRITION & FOOD RESEARCH, WILEY - VCH VERLAG, WEINHEIM, DE, Bd. 52, Nr. 4, 1. April 2008 (2008-04-01), Seiten 483-489, XP002514157 ISSN: 1613-4125

## Beschreibung

Die vorliegende Erfindung betrifft eine Lösung sowie eine Tinktur.

Es sind Cremes bekannt, die sowohl Aloe-Vera als auch Propolis enthalten. Diese Cremes können in ihrer Zusammensetzung jedoch nicht in der Mundhöhle und dem Rachenraum verwendet werden.

Darüber hinaus sind zahlreiche Mundspüllösungen bekannt, die Aloe-Vera enthalten. Auch ist ein Rachenspray bekannt, welches Manukahonig und Propolis sowie ätherische Öle enthält.

So ist aus WO2006/109344 eine Lösung vorbekannt, die u.a. Propolis und Aloe Vera enthält. In dem Artikel "Comparing the saftey, tolerability and efficacy of biotene mouthwash, aloe vera and manuka hoey juice fort he prophylaxis of oral mucositis" von S.K. Tan, S.M. Tan und K.M. Chang, Bone Marrow Transplantation, Bd. 41, nr. 1 Suppl. 1, März 2008 (2008-03), Seite 402, ISSN:0268-3369 ist eine Lösung bekannt, die die Bestandteile Aloe Vera mit aktivem Manukahonig enthält.

In dem Artikel von Rosendale Douglas i et al "High-throughput microbial bisassäys to screen potential New zealand functional food ingredients intended to manage the growth of probiotic and pathogenic gut bacteria", International Journal of food science & Technology, Bd. 43, Nr. 12, Dez. 2008 (2008-12), Seiten 2257-2267, ISSN: 0950-5423 wird das Wachstum von Bakterienkulturen beschrieben bei die mit Lösungen behandelt worden sind, die u.a. die Bestandteile Manukahonig und Propolis aufweisen.

Der von Mavric Elvira et al verfasste Artikel "Identification and quantification of methylglyoxal as the dominant antibacterial constituent of Manuka (Leptospermum scoparium) honeys from New Zealand", Molecular Nutrition & Food Research, Wiley - VCH Verlag, Weinheim, DE, Bd. 52, Nr. 4, 1. April 2008 (2008-4-1), Seiten 483-489, XP002514157 ISSN: 1613-4125, beschreibt die Wirkungsweise von Manukahonig im Allgemeinen.

Aufgabe der vorliegenden Erfindung ist es, eine flüssige Lösung oder Tinktur bereitzustellen, die für die Behandlung des Übergangsgewebes, z.B. der Lippenhaut zu den Mundschleimhäuten hin sowie der Schleimhäute geeigneter ist.

Diese Aufgabe wird erfinderisch mit einer Lösung oder einer Tinktur mit den Merkmalen des Patentanspruchs 1 gelöst. Vorteilhafte Ausgestaltungen der erfindungsgemäßen Lösung sowie der Tinktur ergeben sich durch die Merkmale der Unteransprüche.

Die Erfindung betrifft eine Lösung oder Tinktur mit den Bestandteilen Propolis, Aloe-Vera und Manukahonig. Es hat sich gezeigt, dass gerade durch das Zusammenwirken aller drei Komponenten eine besonders gute Heilung und Prävention des Übergangsgewebes, z.B. der Lippenhaut zu den Mundschleimhäuten hin sowie der Schleimhäute erzielt wird. Propolis kann dabei als Bienenkittharz, in pulvriger oder in flüssiger Form verwendet werden. Aloe-Vera kann als Grundsubstanz in gelartiger oder in flüssiger Form Verwendung finden.

Die erfindungsgemäße Lösung, welche insbesondere eine Mundspüllösung oder Tinktur ist, kann auf die Haut und Mundschleimhäute aufgetragen werden. In der Praxis wird die Lösung entweder durch die Zahnzwischenräume durchgezogen oder gespült. Die Tinktur wird bevorzug mittels Watteträger aufgetragen. Zusätzlich kann die Lösung oder Tinktur leicht einmassiert werden. Eine Dauer von ca. 20 Sekunden reicht meistens aus.

Hierdurch werden aufgrund der im Aloe-Vera-Saft oder Gel enthaltenen Stoffe, wie z.B. Acemannan, die Makrophagen (Fresszellen), die Monozyten, die Antikörper und die T-Killerzellen aktiviert.

Das enthaltene Propolis bewirkt durch die enthaltenen Bioflavonoiden (Vitamin P) eine schmerzlindernde Wirkung im Gewebe, hemmt Entzündungen und bindet Giftstoffe. Zudem weist Propolis eine antibakterielle und antivirale Wirksamkeit auf.

Der Manukahonig dient zur Heilung von kleinen Irritationen der Mundhöhle bzw. der Mundschleimhäute.

Die Kräftigung des Immunsystems ist die Folge der erfindungsgemäßen Lösung oder Tinktur. Zudem wird das Gewebe gestrafft und eine Verbesserung der Abwehrlage auf natürliche Weise durch Anregung der Eigenabwehr erzielt. Auch dienen die erfindungsgemäße Lösung und Tinktur dem Schutz und zur Vorbeugung gegen Zahnfleischentzündung und Parodontose.

Erfindungsgemäß ist die Lösung flüssig und kann zur Tinktur hin eine zähe bis dickflüssige Konsistenz aufweisen.

Vorzugsweise kann der Aloe-Vera Saft bzw. das Aloe-Vera Gel aus der Sorte Barbardensis Miller gewonnen sein.

Es hat sich als besonders vorteilhaft herausgestellt, wenn die Lösung als Basis eine Flüssigkeit, wie insbesondere Öl, besonders bevorzugt Sonnenblumenöl, aufweist. Mit der Basis wird zuerst der Aloe-Vera Saft oder das Aloe-Vera-Gel gemischt. Das Propolis wird zunächst in Wasser, Alkohol oder Öl gelöst und dann mit dem Gemenge aus Öl und Aloe-Vera vermengt bzw. vermischt. Anschließend wird reiner oder gelöster Manukahonig beigegeben.

Eine besonders vorteilhafte Tinktur ergibt sich durch die nachfolgende Rezeptur:
30 ml 30%-ige Propolis-Lösung
20 ml Aloe-Vera 10-fach Konzentrat
1 Teelöffel Manukahonig

Eine 30%-ige Propolis-Lösung ist dabei eine Mischung von 3 Teilen Propolis-Kittharz in 7 Teilen Alkohol. Aloe-Vera 10-fach Konzentrat ist dabei eine handelsüblich verfügbare Lösung.

Vorteilhaft kann die Tinktur auch folgende Substanzanteile aufweisen:
6 bis 60 ml 30%-ige Propolis-Lösung
6 bis 60 ml Aloe-Vera 10-fach Konzentrat
0,2 bis 2 Teelöffel Manukahonig

Zur Herstellung der Tinktur wird zunächst die Propolis-Lösung mit dem Aloe-Vera Konzentrat vermischt. Anschließend wird der Manukahonig beigemischt.

Eine besonders bevorzugte Lösung ergibt sich durch die nachfolgende Rezeptur:
100 ml Öl, insbesondere Sonnenblumenöl
100 ml 30%-ige Propolis-Lösung
450 ml Aloe-Vera 10-fach Konzentrat
50 ml Ethanol 70%-ige Lösung
90 g Manukahonig welcher in 500ml H₂O gelöst ist
3000 ml H₂O

Vorteilhaft kann die Lösung auch folgende Substanzanteile aufweisen:
30 bis 300 ml Öl, insbesondere Sonnenblumenöl
30 bis 300 ml 30%-ige Propolis-Lösung
45 bis 1000 ml Aloe-Vera 10-fach Konzentrat
20 bis 150 ml Ethanol 70%-ige Lösung
10 bis 500 g Manukahonig welcher in 500ml H₂O gelöst ist
1000 bis 3000 ml H₂O

Zur Herstellung der Lösung wird vorteilhaft zunächst das Öl mit dem Aloe-Vera-Konzentrat vermischt. Danach wird die Propolis-Lösung mit dem Ethanol vermischt. Im nachfolgenden Schritt werden die beiden zuvor hergestellten Mischungen miteinander vermischt. Danach wird der in Wasser gelöste Manukahonig der Mischung beigegeben. Als letztes wird die Lösung durch Zugabe von Wasser verlängert bzw. gestreckt.

Sofern anders konzentrierte Ausgangsstoffe verwendet werden, sind die Mischungsverhältnisse entsprechend anzupassen.

Weiterhin kann die Lösung oder die Tinktur als weitere Bestandteile Stoffe wie Teebaumöl, Schafgabe, Citronella, Thymian, Minzöl, L-Carnosin und Manukahonig zur Wirkstoffverstärkung enthalten.

Ebenso können Geschmackskorregenzien zugesetzt werden, um den Geschmack der Tinktur oder Lösung zu verbessern.

## Patentansprüche

1. Lösung oder Tinktur, insbesondere zur Behandlung des Übergangsgewebes von der Lippenhaut zu den Mundschleimhäuten hin sowie der Schleimhäute selbst, **dadurch gekennzeichnet, dass** die Lösung oder Tinktur mindestens Propolis, Aloe-Vera und Manukahonig enthält.

2. Lösung oder Tinktur nach Anspruch 1, **dadurch gekennzeichnet, dass** das Volumenverhältnis 1:10 (Aloe-Vera zu Propolis) oder 1:4 bis 1:1 beträgt.

3. Lösung oder Tinktur nach Anspruch 1, **dadurch gekennzeichnet, dass** das Volumenverhältnis höchstens 10:1 (Aloe-Vera zu Propolis), insbesondere 4:1 bis 2:1 beträgt.

4. Lösung oder Tinktur nach einem der vorhergehenden Ansprüche, **dadurch gekennzeichnet, dass** die Lösung oder Tinktur ein 10-fache Konzentrat Aloe-Vera enthält oder das Volumenverhältnis Aloe-Vera zu Propolis wenigstens 1:100 ist.

5. Lösung oder Tinktur nach einem der vorhergehenden Ansprüche, **dadurch gekennzeichnet, dass** die Lösung oder Tinktur eine flüssige oder zäh- bis dickflüssige Konsistenz aufweist.

6. Lösung oder Tinktur nach einem der vorhergehenden Ansprüche, **dadurch gekennzeichnet, dass** der Aloe-Vera-Saft oder das beigefügte Aloe-Vera-Gel aus der Sorte Barbadensis Miller gewonnen ist.

7. Lösung nach einem der vorhergehenden Ansprüche, **dadurch gekennzeichnet, dass** die Lösung eine Mundspüllösung ist.

8. Lösung nach einem der vorhergehenden Ansprüche, **dadurch gekennzeichnet, dass** die Lösung eine Mischung aus
30 bis 300 ml Öl, insbesondere Sonnenblumenöl,
30 bis 300 ml 30%-ige Propolis-Lösung,
45 bis 1000 ml Aloe-Vera 10-fach Konzentrat
20 bis 150 ml Ethanol 70%-ige Lösung
10 bis 500 g Manukahonig welcher in 500ml H₂O gelöst ist
1000 bis 3000 ml H₂O
ist, insbesondere eine Mischung aus
100 ml Öl, insbesondere Sonnenblumenöl
100 ml 30%-ige Propolis-Lösung
450 ml Aloe-Vera 10-fach Konzentrat
50 ml Ethanol 70%-ige Lösung
90 g Manukahonig welcher in 500ml H₂O gelöst ist
3000 ml H₂O
ist.

9. Tinktur nach einem der vorhergehenden Ansprüche,
**dadurch gekennzeichnet, dass** die Tinktur eine Mischung aus
6 bis 60 ml 30%-ige Propolis-Lösung
6 bis 60 ml Aloe-Vera 10-fach Konzentrat
0,2 bis 2 Teelöffel Manukahonig,
ist, insbesondere eine Mischung aus
30 ml 30%-ige Propolis-Lösung,
20 ml Aloe-Vera 10-fach Konzentrat und
einem Teelöffel Manukahonig
ist.

10. Lösung oder Tinktur nach einem der vorhergehenden Ansprüche, **dadurch gekennzeichnet, dass** weitere Bestandteile wie Teebaumöl, Schafgabe, Citronella, Thymian, Minzöl, und L-Carnosin zur Wirkstoffverstärkung enthalten sind.

11. Zusammensetzung einer Lösung oder Tinktur nach einem der vorhergehenden Ansprüche zur Verwendung bei der Behandlung der Mundhöhle, insbesondere der Zahnfleischsaum und/oder Zahnfleischtaschen und/oder der Mundschleimhaut, insbesondere zur Spülung oder zum Betupfen der Mundhöhle, des Zahnfleischsaums, der Zahnfleischtaschen und/oder der Mundschleimhaut.

12. Herstellungsverfahren zur Herstellung einer Tinktur nach einem der vorhergehenden Ansprüche, **dadurch gekennzeichnet, dass** zunächst die Propolis-Lösung mit dem Aloe-Vera Konzentrat vermischt und anschließend der Manukahonig beigemischt wird.

13. Herstellungsverfahren zur Herstellung einer Lösung nach einem der vorhergehenden Ansprüche 1 bis 12, **dadurch gekennzeichnet, dass** zur Herstellung der Lösung das Öl mit dem Aloe-Vera-Konzentrat und die Propolis-Lösung mit dem Ethanol vermischt wird, wonach danach beide Mischungen miteinander vermischt werden und anschließend der in Wasser gelöste Manukahonig der Mischung beigegeben und als letztes die Lösung durch Zugabe von Wasser verlängert bzw. gestreckt wird.

## Claims

1. Solution or tincture, in particular for the treatment of the transitional tissue of the lip skin towards the oral mucosae as well as the mucosae themselves, **characterised in that** the solution or tincture contains at least propolis, aloe vera and manuka honey.

2. Solution or tincture according to Claim 1, **characterised in that** the volume ratio amounts to 1:10 (aloe vera to propolis) or 1:4 to 1:1.

3. Solution or tincture according to Claim 1, **characterised in that** the volume ratio amounts to, at the highest, 10:1 (aloe vera to propolis), in particular 4:1 to 2:1.

4. Solution or tincture according to one of the preceding claims, **characterised in that** the solution or tincture contains a 10-times aloe vera concentrate or the volume ratio of aloe vera to propolis is at least 1:100.

5. Solution or tincture according to one of the preceding claims, **characterised in that** the solution or tincture has a fluid or semi-fluid to viscous consistency.

6. Solution or tincture according to one of the preceding claims, **characterised in that** the aloe vera juice or the added aloe vera gel is obtained from the type Barbadensis Miller.

7. Solution according to one of the preceding claims, **characterised in that** the solution is a mouth rinse solution.

8. Solution according to one of the preceding claims, **characterised in that** the solution is a mixture of
30 to 300 ml oil, in particular sunflower oil,
30 to 300 ml 30% propolis solution,
45 to 1000 ml 10-times aloe vera concentrate
20 to 150 ml 70% ethanol solution
10 to 500 g manuka honey which is dissolved in 500 ml H₂O
1000 to 3000 ml H₂O
in particular a mixture of
100 ml oil, in particular sunflower oil,
100 ml 30% propolis solution,
450 ml 10-times aloe vera concentrate
50 ml 70% ethanol solution
90 g manuka honey which is dissolved in 500 ml H₂O
3000 ml H₂O.

9. Tincture according to one of the preceding claims, **characterised in that** the tincture is a mixture of
6 to 60 ml 30% propolis solution
6 to 60 ml 10-times aloe vera concentrate
0.2 to 2 teaspoons manuka honey,
in particular a mixture of
30 ml 30% propolis solution
20 ml 10-times aloe vera concentrate and
one teaspoon manuka honey.

10. Solution or tincture according to one of the preceding claims, **characterised in that** further components such as tea tree oil, yarrow, citronella, thyme, mint oil, and L-carnosine are present for the amplification of active ingredients.

11. Composition of a solution or tincture according to one of the preceding claims for use in the treatment of the oral cavity, in particular of the gum margin and/or gum pockets and/or the oral mucosa, in particular for rinsing or dabbing the oral cavity, the gum margin, the gum pockets and/or the oral mucosa.

12. Production method for the production of a tincture according to one of the preceding claims, **characterised in that** firstly the propolis solution is mixed with the aloe vera concentrate and subsequently the manuka honey is mixed in.

13. Production method for the production of a solution according to one of the preceding Claims 1 to 12, **characterised in that** for the production of the solution, the oil is mixed with the aloe vera concentrate and the propolis solution is mixed with the ethanol, after which both mixtures are then mixed with each other and subsequently the manuka honey that is dissolved in water is added to the mixture and lastly the solution is extended or stretched by the addition of water.

## Revendications

1. Solution ou teinture, en particulier pour le traitement du tissu transitoire des lèvres jusqu'aux muqueuses buccales ainsi que pour le traitement des muqueuses elles-mêmes, **caractérisée en ce que** la solution ou la teinture contient au moins du propolis, de l'aloe vera et du miel de Manuka.

2. Solution ou teinture selon la revendication 1, **caractérisée en ce que** le rapport volumique (aloe vera / propolis) s'élève à 1:10, ou de 1:4 à 1:1.

3. Solution ou teinture selon la revendication 1, **caractérisée en ce que** le rapport volumique (aloe vera / propolis) s'élève au maximum à 10:1, en particulier de 4:1 à 2:1.

4. Solution ou teinture selon une des revendications précédentes, **caractérisée en ce que** la solution ou la teinture contient de l' aloe vera 10 fois concentré ou que le rapport volumique aloe vera / propolis est de au moins de 1:100.

5. Solution ou teinture selon une des revendications précédentes, **caractérisée en ce que** la solution ou la teinture présente une consistance liquide, ou de visqueuse à épaisse.

6. Solution ou teinture selon une des revendications précédentes, **caractérisée en ce que** le jus d'aloe vera ou le gel d'aloe vera ci-joint est du type Barbadensis Miller.

7. Solution selon une des revendications précédentes, **caractérisée en ce que** la solution est une solution bain de bouche.

8. Solution selon une des revendications précédentes, **caractérisée en ce que** la solution consiste en un mélange se composant de
de 30 à 300 ml d'huile, en particulier de l'huile de tournesol,
de 30 à 300 ml d'une solution à 30% de propolis,
de 45 à 1000 ml de aloe vera 10 fois concentré,
de 20 à 150 ml d'une solution à 70 % d'éthanol,
de 10 à 500 g de miel de Manuka, lequel est dissous dans 500ml H₂O
1000 à 3000 ml H₂O
en particulier un mélange se composant de
100 ml d'huile, en particulier de l'huile de tournesol,
100 ml d'une solution à 30% de propolis
450 ml d'aloe vera 10 fois concentré,
50 ml d'une solution à 70 % d'éthanol,
90 g de miel de Manuka lequel est dissous dans 500ml H₂O,
3000 ml H₂O.

9. Teinture selon une des revendications précédentes, **caractérisé en ce que** la teinture est un mélange se composant de
de 6 à 60 ml d'une solution à 30% de propolis,
de 6 à 60 ml d'aloe vera 10 fois concentré,
de 0,2 à 2 cuillères à café de miel de Manuka,
en particulier un mélange se composant de
30 ml d'une solution à 30% de propolis,
20 ml d'aloe vera 10 fois concentré et
une cuillère à café de miel de Manuka.

10. Solution ou teinture selon une des revendications précédentes, **caractérisée en ce que** d'autres composants tels que l'huile d'arbre à thé, achillée millefeuille, citronnelle, thym, essence de menthe, et L-Carnosine sont contenus pour l'amplification du principe actif.

11. Composition d'une solution ou teinture selon une des revendications précédentes à utiliser lors du traitement de la cavité buccale, en particulier du liséré gingival et/ou des poches gingivales et/ou de la muqueuse buccale, en particulier pour rincer ou tamponner la cavité buccale, le liséré gingival et/ou les poches gingivales et/ou la muqueuse buccale.

12. Procédé de fabrication permettant de produire une teinture selon une des revendications précédentes, **caractérisé en ce que** premièrement la solution à base de propolis est mélangée avec le concentré d'aloe vera et ensuite l'on ajoute le miel de Manuka.

13. Procédé de fabrication permettant de produire une solution selon une des revendications précédentes de 1 à 12, **caractérisé en ce que**, pour produire la solution, l'huile est mélangée avec le concentré d'aloe vera et la solution à base de propolis est mélangée avec l'éthanol, après quoi les deux mélanges sont mutuellement mélangés et ensuite le miel de Manuka dissous dans l'eau est ajouté au mélange et en tout dernier la solution est rallongée, respectivement allongée par l'ajout d'eau.
